# EUROPEAN PATENT SPECIFICATION

(11) **EP 0 551 706 B1**
(45) Date of publication and mention of the grant of the patent: **28.02.1996**
(21) Application number: 92308135.0
(22) Date of filing: 08.09.1992
(51) Int. Cl.: A61B 17/22

(54) **Atherectomy cutter with a positive attack angle**
Atherektomie-Schneidevorrichtung mit positivem Angriffswinkel
Couteau d'athérectomie avec angle d'attaque positif

(30) Priority: 13.01.1992 US 820748
(43) Date of publication of application: 21.07.1993
(73) Proprietor: INTERVENTIONAL TECHNOLOGIES INC, San Diego California 92123 (US)
(72) Inventor: Radisch, Herbert R., Jr., San Diego, California 92126 (US)
(74) Representative: Coxon, Philip

(56) References cited:
- EP-A- 0 147 192
- EP-A- 0 348 573
- DE-A- 3 242 341
- US-A- 2 730 101

## Description

### FIELD OF THE INVENTION

The present invention relates generally to devices which remove stenoses from blood vessels. More particularly, the present invention relates to atherectomy devices. The present invention particularly, though not exclusively, relates to cutters for atherectomy devices which cut a bore through atherosclerotic plaque.

### BACKGROUND OF THE INVENTION

Blockage of human arteries is a widespread malady and, as such, represents a significant health concern. Blockages reducing blood flow through the coronary arteries to the heart can cause heart attacks, while blockages reducing blood flow through the arteries to the brain can cause strokes. Similarly, arterial blockages reducing blood flow through arteries to other parts of the body can produce grave consequences in an affected organ or limb.

The build-up of atherosclerotic plaque is a chief cause of blockages reducing arterial blood flow. Consequently, several methods have been introduced to alleviate the effects of plaque build-up restricing the arterial lumen. One such method is a procedure termed angioplasty, which uses an inflatable device positioned in the artery to dilate the lumen at the stenosis. A typical angioplasty device is disclosed in U.S. Patent No. 4,896,669 to Bhate et al. The angioplasty device of Bhate et al includes an inflatable balloon which is attached to the distal end of a hollow catheter. The proximal end of the catheter is attached to a fluid source, providing fluid communication between the balloon and the fluid source.

To treat an arterial stenosis, the Bhate et al balloon is introduced into the artery in a deflated state and guided through the artery over a guide wire to a position adjacent the stenosis. Fluid from the fluid source is then infused into the balloon via the catheter to inflate the balloon. As the balloon expands, it dilates the lumen of the artery. The balloon is then deflated and removed from the artery.

While effective for dilating the lumen at the stenosis, angioplasty devices such as the Bhate et al device do not remove the plaque from the artery. Consequently, the residual plaque fragments either remain in place at the point of the stenosis or break off and migrate to other locations in the blood stream. In either case the plaque remains a continuing threat to create blockages in the circulatory system. To address the shortcomings of angioplasty, a procedure termed atherectomy has been devised which cuts a stenosis and removes the plaque fragments comprising the stenosis from the blood vessel.

An atherectomy procedure typically includes inserting a guide wire into the affected artery and advancing a hollow cutting device over the wire until the cutting device is positioned adjacent the stenosis. The cutting device is then advanced into the stenosis to cut a channel through the plaque, thereby increasing blood flow through the artery. The resulting plaque fragments are removed from the blood stream by drawing them into the hollow cutting device.

A number of atherectomy devices capable of performing this procedure are known in the art. U.S. Patent No. 4,887,613 to Farr et al, which is assigned to the same assignee as the present invention and is incorporated herein by reference, discloses an atherectomy device having a frustum-shaped cutter which is attached to the distal end of a hollow catheter. The cutter has a plurality of openings that define cutting blades. The cutter is directed through the artery and rotated as it advances into the stenosis, thereby cutting the plaque. Excised plaque enters the openings of the cutter and is subsequently removed through the hollow catheter.

While the Farr et al device is at least somewhat effective for its intended purpose, due to the configuration of the cutter blades, the orientation of the blades relative to the surface of the plaque being cut is less than optimal. This encumbers the cutting procedure and may result in unacceptable amounts of plaque remaining in the artery which can ultimately lead to a restenosis of the affected artery. Accordingly, the present invention recognizes a need to provide an atherectomy device which more effectively cuts a smooth channel through a stenosis than known devices.

It is therefore an object of the present invention to provide an atherectomy cutter that can cut a channel through a stenosis in an artery of a living being. Another object of the present invention is to provide an atherectomy cutter that cuts a relatively smooth and wide channel through the arterial stenosis. Yet another object of the present invention is to provide an atherectomy cutter that minimizes the residual plaque remaining in the artery as the channel is cut. Finally, it is an object of the present invention to provide an atherectomy cutter which is relatively easy to use and cost-effective to manufacture.

The present invention is defined in the accompanying claims with the features known from EP-A-348573 in the precharacterising portion. intersect at the longitudinal axis of the cutter. The blade elements are fixably connected to one another at their point of intersection to define a hub. The hub has an opening extending through it which enables positioning of the cutter on a guide wire.

Each blade element of the cutter is a relatively rigid band having an outer face that is characterized as a flat planar surface facing away from the longitudinal axis of the cutter. The outer face has two linear and substantially longitudinal edges, extending between the proximal and distal ends of the blade element. One edge defines the cutting edge of the blade element and the other defines the trailing edge. As the blade element is rotated about the longitudinal axis of the cutter, the leading edge is termed the cutting edge while the edge following the cutting edge in the direction of rotation is termed the trailing edge.

According to one embodiment of the cutter, the outer face of the blade element is oriented relative to the longitudinal axis of the cutter such that, for any given point on the axis, the radial distance from the axis to the cutting edge and the radial distance from the axis to the trailing edge are substantially equal. Further, the outer face is oriented such that the radial distance from the axis to the cutting or trailing edge is greater than the radial distance from the axis to any other longitudinally corresponding point lying on the outer face of the blade element. Stated differently, rotationally sweeping the cutting and trailing edges of the blade element about the longitudinal axis of the cutter defines a continuous frustum-shaped cutting surface which is the maximum or outermost rotation surface of the blade element.

According to another embodiment of the cutter, the blade element of the previous embodiment is reoriented by rotating it slightly about its own longitudinal axis to a new position relative to the longitudinal axis of the cutter. As a result, for any given point on the longitudinal axis of the cutter, there is only one maximum radial point on the outer face of the blade element and this point lies on the cutting edge. The trailing edge, in distinction contains the minimum radial point on the outer face. Thus, the cutting edge exclusively defines the outermost rotation surface of the blade element.

According to either embodiment of the cutter described above, the proximal end of the cutter is attachable to the distal end of a hollow catheter. By engaging a rotational drive motor with the proximal end of the catheter, the catheter can be rotated. As is apparent, rotation of the catheter is translated into rotation of the cutter. Coupling of the cutter and associated catheter also provides a continuous passageway for cuttings in a proximal direction away from the blade elements.

The cutter of the present invention is operated by positioning it on the distal end of the catheter and inserting it through the skin of a patient into a stenotic artery to be treated. The cutter is guided through the artery, typically along a guide wire previously inserted into the artery, to the stenosis. The cutter is then urged into the stenosis while rotating the catheter and cutter. The rotating cutter is driven through the entire stenosis, cutting a smooth wide channel therethrough which preferably extends to the artery wall.

Because of the blade element configurations described above, contact between the blade elements and the plaque making up the stenosis is primarily limited to those portions of each blade element which are at a maximum radial distance from the longitudinal axis of the cutter, i.e., either the cutting edge alone, or the cutting and trailing edges simultaneously. A clearance is maintained between the stenosis and the remaining portions of the outer face which are interior to the edges.

The degree of clearance between the stenosis and the outer face of the blade element interior to the edges can be characterized by what is termed the attack angle, i.e., the angle between the outer face and the stenotic surface behind the cutting edge of the blade element. In general increasing the attack angle increases the clearance, thereby enabling the cutting edge to more aggressively attack and cut the plaque making up the stenosis. This effect can be attributed to the fact that a clearance prevents the outer face from diminishing the force of the cutting edge on the plaque.

As the plaque is cut away from the stenosis, the cuttings can be collected in the distal end of the hollow catheter and proximally displaced therethrough. When a satisfactory channel is completed through the stenosis, the cutter and catheter containing the cuttings are withdrawn back out through the artery, after which the patient is allowed to recover.

The features of this invention, as well as the invention itself, both as to its structure and its operation, will be best understood from the accompanying drawing, taken in conjunction with the accompanying description, in which similar reference characters refer to similar parts.

### BRIEF DESCRIPTION OF THE DRAWINGS

Figure 1 is a schematic view of the atherectomy cutter of the present invention in its intended operational environment.

Figure 2 is a perspective view of the atherectomy cutter of the present invention.

Figure 3A is a cross-sectional view of the atherectomy cutter of the present invention as seen along line 3-3 in Figure 2 showing the blade elements.

Figure 3B is a cross-sectional view of the atherectomy cutter of the present invention showing a further embodiment of the blade elements.

Figure 3C is a cross-sectional view of the atherectomy cutter of the present invention showing another embodiment of the blade elements.

Figure 3D is a cross-sectional view of the atherectomy cutter of the present invention showing yet another embodiment of the blade elements.

Figure 4 is a schematic cross-sectional view of a prior art atherectomy cutter in the operational environment of an arterial stenosis.

Figure 5 is a schematic cross-sectional view of the atherectomy cutter of the present invention in the operational environment of an arterial stenosis.

### DESCRIPTION OF PREFERRED EMBODIMENTS

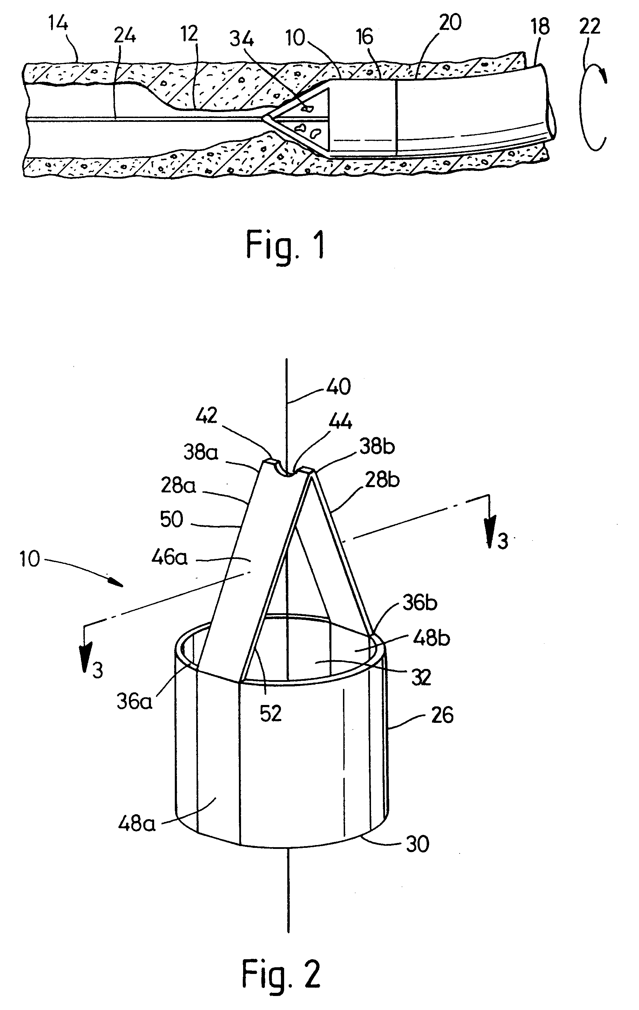

Referring initially to Figure 1, an atherectomy cutter generally designated **10** is shown cutting a substantially smooth channel through a stenosis **12** that is formed on the interior wall of an artery **14**. As shown, the proximal end **16** of cutter **10** is attached to a hollow rotatable catheter **18** at its distal end **20**. Catheter **18** is any suitable tubular structure which can transmit torque, e.g., in the direction indicated by clockwise arrow **22**, from a motor (not shown) to cutter **10**. Cutter **10** can be steered by an operator to the stenosis **12** within artery **14** over a flexible guide wire **24** that extends through torque tube **18** and atherectomy cutter **10**.

The materials of atherectomy cutter assembly **10** are preferably lightweight and strong, as well as chemically inert with the tissue of artery **14**. For example, atherectomy cutter **10** can be made of 400 series stainless steel. Similarly, guide wire **24** can be a flexible yet strong stainless steel wire.

The details of atherectomy cutter **10** are best seen with cross-reference to Figures 1 and 2. Cutter **10** comprises a body **26** and a pair of opposing blade elements **28a**, **28b** integral therewith. Body **26** is hollow and has a proximal opening **30** and a distal opening **32**. Body **26** is substantially cylindrical in shape to enable coupling with catheter **18** as shown in Figure 1. With body **26** coaxially positioned in an interference fit with catheter **18**, body **26** provides a continuous passageway from blade elements **28a**, **28b** into catheter **18**. If desired, catheter **18** and body **26** can further be epoxy-bonded or spot welded together.

As shown in Figure 1, cuttings **34** from stenosis **12**, are permitted to flow through the passageway formed by body **26** and through catheter **18** for withdrawal from artery **14**. Passage of cuttings **34** through catheter **18** may be facilitated by providing a suction means (not shown) at the proximal end of catheter **18** in a manner known to one skilled in the art.

Returning to Figure 2, proximal ends **36a**, **36b** of blade elements **28a**, **28b** are peripherally and opposingly mounted around distal opening **32**. Blade elements **28a**, **28b** extend along a linear pathway from body **26**. The direction of extension has both a distally longitudinal and an inwardly radial component causing convergence of blade elements **28a**, **28b** at their distal ends **38a**, **38b** on the longitudinal axis **40** of cutter **10**. Thus, blade elements **28a**, **28b** are obliquely aligned relative to axis **40**. The intersection of distal ends **38a**, **38b** defines a hub **42**, at which blade elements **28a**, **28b** are attached to one another. Hub **42** has an opening **44** formed through it to receive guide wire **24**.

Blade elements **28a**, **28b** are substantially identical in configuration. Accordingly both will be described hereafter with reference to a single blade element **28a**, it being understood that the description applies likewise to blade element **28b**. Blade element **28a** is configured as a straight band that preferably is relatively rigid. As such, blade element **28a** has an outer face **46a** that is a flat trapezoidal-shaped outer planar surface facing away from the longitudinal axis **40** of cutter **10**. The flat surface of outer face **46a** can be continuous with body **26**, thereby providing flattened portions **48a**, **48b** on substantially cylindrical body **26**.

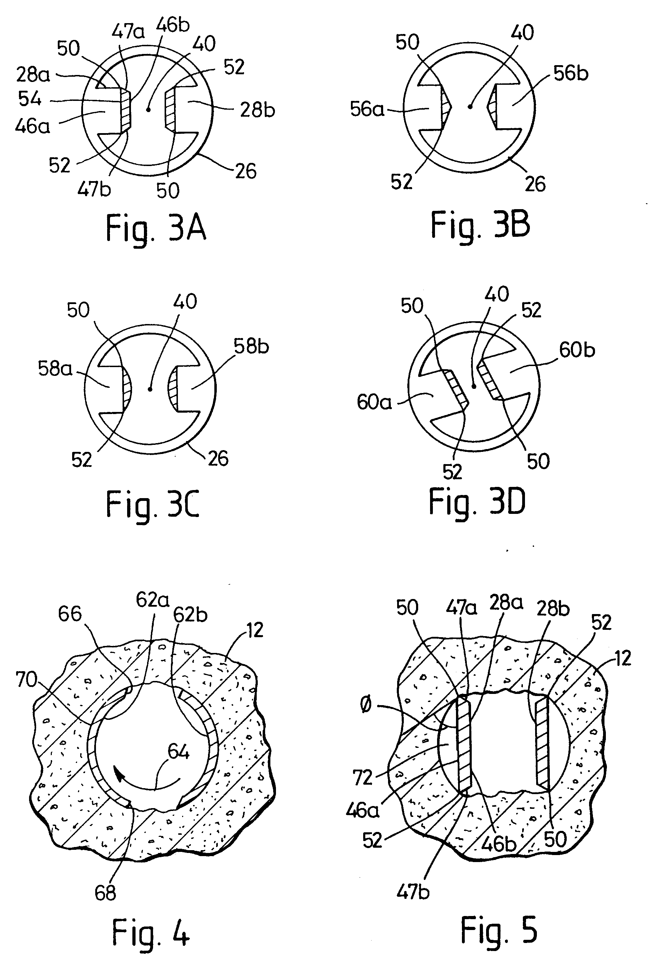

Blade element **28a** further has an inner face **46b** shown in Figure 3A that is a flat trapezoidal-shaped inner planar surface facing toward the longitudinal axis **40** of cutter **10**. Inner face **46b** and outer face **46a** are aligned parallel to one another, and inner face **46b** has a width less than outer face **46a**. First and second side surfaces **47a**, **47b** are positioned between inner and outer faces **46a**, **46b** respectively.

The intersection of outer face **46a** with first and second side surfaces **47a**, **47b** defines two linear edges **50**, **52**, respectively which are longitudinally exposed and aligned in an oblique direction corresponding to the extension of blade element **28a**. When cutter **10** is operated in the clockwise direction of rotation, edge **50** is the cutting edge of blade element **28a** and edge **52** is the trailing edge. The function of edges **50**, **52** can be reversed in a manner apparent to the skilled artisan, if cutter **10** is desired to be operable in the counterclockwise direction of rotation.

The specific orientation of outer face **46a** relative to the longitudinal axis **40** of cutter **10** is best seen with reference to Figures 3A-3D and Figure 5. Referring initially to Figure 3A, the longitudinal axis **40** of cutter **10** is shown in cross-section as a single central point within cutter **10**. It is apparent that the radial distance from axis **40** to cutting edge **50** and the radial distance from axis **40** to trailing edge **52** are equal, and further that this distance is a maximum, i.e., greater than the radial distance from axis **40** to any other longitudinally corresponding point lying on outer face **46a** of blade element **28a**, e.g., point **54**. Consequently, cutting edge **50** and trailing edge **52** define the maximum or outermost rotation surface of blade element **28a**.

As is further apparent from Figure 3A, blade element **28a** has the configuration of a trapezoidal solid, wherein outer face **46a** forms the base of the trapezoidal solid. It is apparent, however, from Figures 3B and 3C that the blade element can have alternate configurations and fall within the scope of the present invention. In Figure 3B, blade elements **56a**, **56b** have the configuration of a triangular solid and in Figure 3C blade elements **58a**, **58b** have the configuration of a semi-cylindrical solid.

The blade element configurations shown in Figures 3B and 3C both satisfy the requirement that the radial distance from the longitudinal axis to the cutting and trailing edges is greater than the radial distance from the axis to any other longitudinally corresponding point lying on the outer face of the blade element. Consequently, as in Figure 3A, the blade elements embodied in Figures 3B and 3C have cutting and trailing edges that define the outermost rotation surface of the blade elements.

Figure 3D shows an embodiment of cutter **10**, wherein blade elements **60a**, **60b** have a similar configuration to blade elements **28a**, **28b** of Figure 3A, but are rotated somewhat about their own longitudinal axes, thereby displacing cutting edge **50** away from the longitudinal axis **40** of cutter **10** while displacing trailing edge **52** toward axis **40**. Consequently, the radial distance from longitudinal axis **40** to any longitudinally corresponding point on outer face **46a** is a maximum at cutting edge **50** and a minimum at trailing edge **52**, and cutting edge **50** alone defines the outermost rotation surface of blade elements **60a**, **60b**.

It is understood that blade elements **56a**, **56b** and **58a**, **58b** of Figures 3B and 3C respectively, can be rotated in a manner similar to that shown in Figure 3D, and that such embodiments fall within the scope of the present invention.

### OPERATION

In the operation of atherectomy cutter **10**, reference is initially made to Figure 1. In accordance with well-known surgical techniques, guide wire **24** is positioned within artery **14**. Catheter **18** having atherectomy cutter **10** attached thereto is advanced over wire **24** until cutter **10** is positioned adjacent stenosis **12**. Catheter **18** is then rotated causing cutter **10** to rotate. As cutter **10** rotates, it is advanced into stenosis **12** where plaque is encountered. Accordingly, cutter **10** progresses forward to cut a wide and substantially smooth channel therethrough. Plaque cuttings **34** which are excised are drawn into catheter **18** by applying a vacuum thereto. Upon completion of the channel, cutter **10** is withdrawn back out through artery **14**.

The enhanced plaque cutting ability of cutter **10** is best demonstrated by comparing the operation of cutter **10** with a prior art cutter configuration. Referring to Figure 4, a pair of prior art blade elements **62a**, **62b**, such as those defined by the frustum-shaped cutter of U.S. Patent No. 4,887,613, are shown in schematic operation within stenosis **12**. For purposes of illustration, only one blade element **62a** is described below since blade elements **62a** and **62b** are substantially identical. It is understood that the description of blade element **62a** applies likewise to blade element **62b**.

Blade element **62a** is rotated in a clockwise direction designated by arrow **64**, which differentiates cutting edge **66** from trailing edge **68**, edges **66**, **68** being separated by a curved outer face **70**. When blade element **62a** enters stenosis **12** as shown, the entire outer face **70** contacts stenosis **12**, not just edges **66**, **68**. Upon such contact, stenosis **12** is compressed both ahead and behind cutting edge **66** due to the curvature of outer face **70** and the elastic nature of the plaque. The attack angle between blade element **62a** and stenosis **12** is essentially zero. Consequently, stenosis **12** is only partially exposed ahead of cutting edge **66** for cutting thereby.

As blade element **62a** is withdrawn from stenosis **12**, however, the compression force on stenosis **12** by outer face **70** diminishes, and stenosis **12** undesireably returns to an expanded diameter. Thus, it is apparent that removal of a stenosis with blade elements lacking a positive attack angle is less effective because much of the stenosis remains in place after treatment.

Referring to Figure 5, the schematic operation of blade elements **28a**, **28b**, as previously shown in Figure 2, is demonstrated in accordance with the present invention. Contact between blade elements **28a**, **28b** and stenosis **12** is essentially limited to cutting edges **50** and trailing edges **52**, while a clearance **72** is maintained between stenosis **12** and the remainder of outer face **46a**. Clearance **72** is characterized by attack angle φ which is the angle between outer face **46a** and the tangent to the stenotic surface behind cutting edge **50**. As shown, φ has a positive value typically on the order of about 45°. The positive attack angle avoids compression of stenosis **12** by outer face **46a** and fully exposes stenosis **12** to cutting edge **50**, thereby enabling effective cutting of a wide smooth channel through stenosis **12**.

While the particular atherectomy cutter as herein shown and disclosed in detail is capable of obtaining the objects and providing the advantages hereinbefore stated, it is understood that this particular cutter is merely illustrative of presently preferred embodiments of the invention. It is further understood that the present invention is not intended to be so limited and that other embodiments are further possible within the scope of the present invention.

For example, although the cutter of the present invention has been described above with reference to a pair of blade elements, it is understood that the present invention may also apply to a cutter having any number of blade elements, such as can be constructed by the skilled artisan employing the teaching set forth herein.

## Claims

1. A rotatable atherectomy cutter (10) having a longitudinal axis and positionable within an artery for removing a stenosis therefrom, said cutter comprising:
a body (26) having a proximal end (16) and a distal end;
at least one rigid blade element (28a,28b) having a proximal end (36a,36b) and a distal end (38a,38b), said proximal end (36a,36b) of said blade element (28a,28b) peripherally mounted on said distal end of said body (26) and linearly extending from said body (26) in a distal direction convergent with the longitudinal axis (40) of said cutter (10)
said blade element (28a,28b) further has an outer face (46a) directed away from the longitudinal axis (40) of said cutter (10) and bounded on opposite sides by a first cutting edge (50) and a second edge (52) extending between said proximal (36a,36b) and distal ends (38a,38b) of said blade element (28a,28b);
said blade element (28a,28b) further having a first flat side surface (47a) and a second flat side surface (47b) characterised in that: said blade element (28a,28b) has a flat planar outer face and said flat side surfaces (47a,47b) extend respectively from said first (50) and second (52) edges toward said longitudinal axis (40), each of said side surfaces (47a,47b) forming an acute angle between said side surface (47a,47b) and said planar outer face (46a), with the result that the radial distance from the longitudinal axis (40) and the first edge (50) is greater than the radial distance from the longitudinal axis (40) to any other longitudinally corresponding point lying on the outer face (46a), so said first cutting edge (50) establishes a positive attack angle between said planar outer face (46a) and a surface of said stenosis during rotation of said cutter (10).

2. A rotatable atherectomy cutter (10) according to Claim 1 further comprising a plurality of said blade elements (28a,28b).

3. A rotatable atherectomy cutter (10) according to Claim 1 or 2 wherein said body (26) is substantially cylindrical and hollow, having proximal and distal openings (30,32), and further wherein said proximal end (36a,36b) of said blade element (28a,28b) is diametrically mounted at said distal opening (32).

4. A rotatable atherectomy cutter (10) according to Claim 2 comprising a pair of blade elements (28a,28b) peripherally and opposingly mounted around said distal opening (32).

5. A rotatable atherectomy cutter (10) according to Claim 4 wherein the distal ends (38a,38b) of the blade elements (28a,28b) are fixably connected to each other at the longitudinal axis (40) of the cutter (10).

6. A rotatable atherectomy cutter (10) according to any one of the preceding claims wherein said proximal end (16) of said body engages a hollow rotatable catheter (18).

7. A rotatable atherectomy cutter (10) according to Claim 6 wherein said body (26) forms a continuous passageway from said blade element (28a,28b) to said hollow catheter (18).

8. A rotatable atherectomy cutter (10) according to any one of the preceding claims wherein said first edge (50) and said second edge (52) are substantially parallel.

9. A rotatable atherectomy cutter (10) according to any one of the preceding claims wherein said blade element (28a,28b) has a trapezoidal cross-section with said outer face (46a) defining the base of said trapezoidal cross-section and said first and second edges (50,52) defining the corners of said base.

10. A rotatable atherectomy cutter (10) according to any one of the preceding claims wherein said first and second edges (50,52) are radially equidistant from the longitudinal axis (40) of said cutter (10).

11. A rotatable atherectomy cutter (10) according to Claim 10 wherein second edge (52) follows said maximum rotation surface upon rotation of said blade element (28a,28b) about the longitudinal axis (40) of said cutter (10).

12. A rotatable atherectomy cutter (10) according to Claim 10 wherein the radial distance from the longitudinal axis (40) of said cutter (10) to said first and second edges (50,52) is greater than the radial distance from said longitudinal axis (40) to any other longitudinally corresponding point on said outer face (46a).

13. A cutting element for use with an atherectomy system to remove plaque from a stenotic segment in an artery which comprises:
a blade element (28a,28b) having a proximal end (36a,36b), a distal end (38a,38b);
said blade element (28a,28b) being connected to a body (26) having an axis of rotation (40);
characterised by:
said blade element (28a,28b) having a trapezoidal shaped cross section being defined by outer and inner flat planar surfaces (46a,46b) with first (47a) and second (47b) flat planar side surfaces therebetween, said inner surface (46b) being of less width than said outer surface (46a) and being oriented relative to said outer surface (46a) to establish a first cutting edge (50) where said first side surface (47a) intersects said outer surface (46a), with the result that the radial distance from the longitudinal axis (40) to the first edge (50) is greater than the radial distance from the longitudinal axis (40) to any other longitudinally corresponding point lying on the outer face (46a); and
means for holding said blade element (28a,28b) to position said inner surface (46b) between said outer surface (46a) and said axis of rotation, and to incline said blade element (28a,28b) with said distal end (38a,38b) nearer said axis of rotation than said proximal end (36a,36b).

## Patentansprüche

1. Rotationsfähiges Atheromektomie-Schneidgerät (10), das eine Längsachse aufweist und in einer Artrie zum Entfernen einer Stenose aus dieser positionierbar ist, welches Schneidgerät umfaßt:
einen Körper (26) mit einem proximalen Ende (16) und einem distalen Ende,
mindestens ein steifes bzw. starres Klingenelement (28a 28b) mit einem proximalen Ende (36a, 36b) und einem distalen Ende (38a, 38b), wobei das proximale Ende (36a, 36b) des Klingenelements (28a, 28b) am Umfang des distalen Endes des Körpers (26) montiert bzw. angebracht ist und vom Körper (26) in einer mit der Längsachse (40) des Schneidgeräts (10) zusammenlaufenden distalen Richtung abgeht,
wobei das Klingenelement (28a, 28b) ferner eine Außenfläche (46a) aufweist, die von der Längsachse (40) des Schneidgeräts (10) hinweg gerichtet und an gegenüberliegenden Seiten durch eine erste Schneidkante (50) und eine zweite Kante (52), die zwischen den proximalen (36a, 36b) und distalen (38a, 38b) Enden des Klingenelements (28a, 28b) verlaufen, begrenzt ist, (und)
wobei das Klingenelement (28a, 28b) weiterhin eine erste flache Seitenfläche (47a) und eine zweite flache Seitenfläche (47b) aufweist,
dadurch gekennzeichnet, daß das Klingenelement (28a, 28b) eine flache, plane Außenfläche aufweist und die flachen Seitenflächen (47a, 47b) jeweils von den ersten (50) und zweiten (52) Kanten in Richtung auf die Längsachse (40) verlaufen und jede der Seitenflächen (47a, 47b) einen spitzen Winkel zwischen der Seitenfläche (47a, 47b) und der planen Außenfläche (46a) festlegt, mit dem Ergebnis, daß das (die) radiale Abstand oder Strecke von der Längsachse (40) zur ersten Kante (50) größer ist als der (die) radiale Abstand oder Strecke von der Längsachse (40) zu jedem anderen, in Längsrichtung entsprechenden, auf der Außenfläche (46a) liegenden Punkt, so daß die erste Schneidkante (50) bei Rotation des Schneidgeräts (10) einen positiven Angreif- oder Anstellwinkel zwischen der planen Außenfläche (46a) und einer Oberfläche der Stenose festlegt.

2. Rotationsfähiges Atheromektomie-Schneidgerät (10) nach Anspruch 1, ferner umfassend mehrere Klingenelemente (28a, 28b).

3. Rotationsfähiges Atheromektomie-Schneidgerät (10) nach Anspruch 1 oder 2, wobei der Körper (26) im wesentlichen zylindrisch und hohl ist und proximale sowie distale Öffnungen (30, 32) aufweist und wobei ferner das proximale Ende (36a, 36b) des Klingenelements (28a, 28b) an der distalen Öffnung (32) diametral montiert ist.

4. Rotationsfähiges Atheromektomie-Schneidgerät (10) nach Anspruch 2, umfassend zwei Klingenelemente (28a, 28b), die umfangsmäßig (beabstandet) und einander gegenüberliegend um die distale Öffnung (32) herum montiert sind.

5. Rotationsfähiges Atheromektomie-Schneidgerät (10) nach Anspruch 4, wobei die distalen Enden (38a, 38b) der Klingenelemente (28a, 28b) an der Längsachse (40) des Schneidgeräts (10) fest (fixably) miteinander verbunden sind.

6. Rotationsfähiges Atheromektomie-Schneidgerät (10) nach einem der vorangehenden Ansprüche, wobei das proximale Ende (16) des Körpers mit einem hohlen, rotationsfähigen Katheter (18) in Eingriff steht.

7. Rotationsfähiges Atheromektomie-Schneidgerät (10) nach Anspruch 6, wobei der Körper (26) einen fortlaufenden Durchgang vom Klingenelement (28a, 28b) zum hohlen Katheter (18) bildet.

8. Rotationsfähiges Atheromektomie-Schneidgerät (10) nach einem der vorangehenden Ansprüche, wobei die erste Kante (50) und die zweite Kante (52) im wesentlichen (zueinander) parallel sind.

9. Rotationsfähiges Atheromektomie-Schneidgerät (10) nach einem der vorangehenden Ansprüche, wobei das Klingenelement (28a, 28b) einen trapezförmigen Querschnitt aufweist, bei dem die Außenfläche (46a) die Basis bzw. Grundlinie des trapezförmigen Querschnitts und die ersten und zweiten Kanten (50, 52) die Ecken der Grundlinie definieren.

10. Rotationsfähiges Atheromektomie-Schneidgerät (10) nach einem der vorangehenden Ansprüche, wobei die ersten und zweiten Kanten (50, 52) radial gleich weit von der Längsachse (40) des Schneidgeräts (10) entfernt sind.

11. Rotationsfähiges Atheromektomie-Schneidgerät (10) nach Anspruch 10, wobei die zweite Kante (52) bei Rotation des Klingenelements (28a, 28b) um die Längsachse (40) des Schneidgeräts (10) der maximalen bzw. äußersten Rotationsfläche folgt.

12. Rotationsfähiges Atheromektomie-Schneidgerät (10) nach Anspruch 10, wobei der (die) radiale Abstand oder Strecke von der Längsachse (40) des Schneidgeräts (10) zu den ersten und zweiten Kanten (50, 52) größer ist als der (die) radiale Abstand oder Strecke von der Längsachse (40) zu einem gegebenen anderen, in Längsrichtung entsprechenden Punkt auf der Außenfläche (46a).

13. Schneidelement bzw. -gerät zur Verwendung bei einem Atheromektomiesystem zum Entfernen von Plaque bzw. Ablagerung von einem stenotischen Segment in einer Arterie, umfassend:
ein Klingenelement (28a, 28b) mit einem proximalen Ende (36a, 36) und einem distalen Ende (38a, 38b),
welches Klingenelement (28a, 28b) mit einem eine Rotationsachse (40) aufweisenden Körper (26) verbunden ist,
dadurch gekennzeichnet, daß
das Klingenelement (28a, 28b) einen durch äußere und innere flache Planflächen (46a, 46b) mit dazwischen liegenden ersten (47a) und zweiten (47b) flachen, planen Seitenflächen definierten trapezförmigen Querschnitt aufweist, wobei die Innenfläche (46b) eine kleinere Breite als die Außenfläche (46a) aufweist und relativ zur Außenfläche (46a) so orientiert ist, daß an der Schnittstelle der ersten Seitenfläche (47a) mit der Außenfläche (46a) eine erste Schneidkante (50) festgelegt ist, mit dem Ergebnis, daß der (die) radiale Abstand oder Strecke von der Längsachse (40) zur ersten Kante (50) größer ist als der (die) radiale Abstand oder Strecke von der Längsachse (40) zu jedem gegebenen anderen, in Längsrichtung entsprechenden, auf der Außenfläche (46a) liegenden Punkt, und
Mittel zum Halten des Klingenelements (28a, 28b) zum Positionieren der Innenfläche (46b) zwischen der Außenfläche (46a) und der Rotationsachse sowie zum Schrägstellen des Klingenelements (28a, 28b), so daß sich sein distales Ende (38a, 38b) näher an der Rotationsachse befindet als das proximale Ende (36a, 36b), vorgesehen sind.

## Revendications

1. Couteau rotatif (10) pour athérectomie, ayant un axe longitudinal, et pouvant être positionné à l'intérieur d'une artère pour supprimer une sténose de celle-ci, ledit couteau comportant :
un corps (26) ayant une extrémité proximale (16) et une extrémité distale,
au moins un élément de lame rigide (28a, 28b) ayant une extrémité proximale (36a, 36b) et une extrémité distale (38a, 38b), ladite extrémité proximale (36a, 36b) dudit élément de lame (28a, 28b) étant montée périphériquement sur ladite extrémité distale dudit corps (26) et s'étendant linéairement à partir dudit corps (26) dans une direction distale en convergeant sur l'axe longitudinal (40) dudit couteau (10)
ledit élément de lame (28a, 28b) comportant de plus une face extérieure (46a) dirigée s'éloignant de l'axe longitudinal (40) dudit couteau (10) et délimitée sur ses côtés opposés par un premier bord de coupe (50) et un second bord (52) s'étendant entre lesdites extrémités proximale (36a, 36b) et distale (38a, 38b) dudit élément de lame (28a, 28b),
ledit élément de lame (28a, 28b) ayant de plus une première surface latérale plate (47a) et une seconde surface latérale plate (47b), caractérisé en ce que ledit élément de lame (28a, 28b) a une face extérieure plane et lesdites surfaces latérales planes (47a, 47b) s'étendent respectivement à partir dudit premier bord (50) et dudit second bord (52) en direction dudit axe longitudinal (40), chacune desdites surfaces latérales (47a, 47b) délimitant un angle aigu entre ladite surface latérale (47a, 47b) et ladite face extérieure plane (46a), le résultat étant que la distance radiale depuis l'axe longitudinal (40) jusqu'au premier bord (50) est plus grande que la distance radiale depuis l'axe longitudinal (40) jusqu'à un autre point quelconque correspondant longitudinalement de la face extérieure (46a), de sorte que ledit premier bord de coupe (50) établit un angle d'attaque positif entre ladite face extérieure plane (46a) et une surface de ladite sténose pendant la rotation dudit couteau (10).

2. Couteau rotatif (10) pour athérectomie, selon la revendication 1, comportant en outre plusieurs éléments de lame (28a, 28b).

3. Couteau rotatif (10) pour athérectomie, selon la revendication 1 ou 2, dans lequel ledit corps (26) est pratiquement cylindrique et creux, ayant des ouvertures proximale et distale (30, 32) et dans lequel en outre ladite extrémité proximale (36a, 36b) dudit élément de lame (28a, 28b) est montée diamétralement au niveau de ladite ouverture distale (32).

4. Couteau rotatif (10) pour athérectomie, selon la revendication 2, comportant une paire d'éléments de lame (28a, 28b) montés périphériquement et de manière opposée autour de ladite ouverture distale (32).

5. Couteau rotatif (10) pour athérectomie, selon la revendication 4, dans lequel les extrémités distales (38a, 38b) des éléments de lame (28a, 28b) sont reliées de manière fixe l'une à l'autre au niveau de l'axe longitudinal (40) du couteau (10).

6. Couteau rotatif (10) pour athérectomie, selon l'une quelconque des revendications précédentes, dans lequel ladite extrémité proximale (16) dudit corps vient en prise avec un cathéter rotatif creux (18).

7. Couteau rotatif (10) pour athérectomie, selon la revendication 6, dans lequel ledit corps (26) forme un passage continu à partir dudit élément de lame (28a, 28b) jusqu'audit cathéter creux (18).

8. Couteau rotatif (10) pour athérectomie, selon l'une quelconque des revendications précédentes, dans lequel ledit premier bord (50) et ledit second bord (52) sont pratiquement parallèles.

9. Couteau rotatif (10) pour athérectomie, selon l'une quelconque des revendications précédentes, dans lequel ledit élément de lame (28a, 28b) a une section transversale trapézoïdale, ladite face extérieure (46a) définissant la base de ladite section transversale trapézoïdale et ledit premier bord et ledit second bord (50, 52) définissant les sommets de ladite base.

10. Couteau rotatif (10) pour athérectomie, selon l'une quelconque des revendications précédentes, dans lequel ledit premier bord et ledit second bord (50, 52) sont radialement équidistants de l'axe longitudinal (40) dudit couteau (10).

11. Couteau rotatif (10) pour athérectomie, selon la revendication 10, dans lequel le second bord (52) suit ladite surface de rotation maximale lors de la mise en rotation dudit élément de lame (28a, 28b) autour de l'axe longitudinal (40) dudit couteau (10).

12. Couteau rotatif (10) pour athérectomie, selon la revendication 10, dans lequel la distance radiale à partir de l'axe longitudinal (40) dudit couteau (10) jusqu'aux premier et second bords (50, 52) est plus grande que la distance radiale depuis ledit axe longitudinal (40) jusqu'à tout autre point quelconque correspondant longitudinalement de ladite surface extérieure (46a).

13. Elément de coupe destiné à être utilisé avec un système d'athérectomie pour supprimer une plaque d'un tronçon sténosé d'une artère, qui comporte :
un élément de lame (28a, 28b) ayant une extrémité proximale (36a, 36b), une extrémité distale (38a, 38b),
ledit élément de lame (28a, 28b) étant relié à un corps (26) ayant un axe de rotation (40),
caractérisé en ce que :
ledit élément de lame (28a, 28b) a une section transversale de forme trapézoïdale définie par des surfaces planes extérieure et intérieure (46a, 46b) ayant des première (47a) et seconde (47b) surfaces latérales planes situées entre elles, ladite surface intérieure (46b) ayant une largeur plus petite que ladite surface extérieure (46a) et étant orientée par rapport à ladite surface extérieure (46a) pour établir un premier bord de coupe (50) où ladite première surface latérale (47a) recoupe ladite surface extérieure (46a), en ayant pour résultat que la distance radiale à partir de l'axe longitudinal (40) jusqu'au premier bord (50) est plus grande que la distance radiale depuis l'axe longitudinal (40) jusqu'à tout autre point quelconque correspondant longitudinalement situé sur la face extérieure (46a), et par
des moyens pour maintenir ledit élément de lame (28a, 28b) pour positionner ladite surface intérieure (46b) entre ladite surface extérieure (46a) et ledit axe de rotation, et pour incliner ledit élément de lame (28a, 28b), ladite extrémité distale (38a, 38b) étant plus proche dudit axe de rotation que ladite extrémité proximale (36a, 36b).
